Europäisches Patentamt

European Patent Office

Office européen des brevets

(11) Veröffentlichungsnummer : **0 351 357 B1**

# (12) EUROPÄISCHE PATENTSCHRIFT

(45) Veröffentlichungstag der Patentschrift :
03.03.93 Patentblatt 93/09

(21) Anmeldenummer : **89810461.7**

(22) Anmeldetag : **15.06.89**

(51) Int. Cl.⁵ : **A01K 61/00, A01K 29/00**

(54) **Verbessertes Fischzuchtverfahren.**

(30) Priorität : **24.06.88 CH 2429/88**

(43) Veröffentlichungstag der Anmeldung :
**17.01.90 Patentblatt 90/03**

(45) Bekanntmachung des Hinweises auf die
Patenterteilung :
**03.03.93 Patentblatt 93/09**

(84) Benannte Vertragsstaaten :
**AT BE CH DE ES FR GB GR IT LI LU NL SE**

(56) Entgegenhaltungen :
**DE-A- 2 213 905
US-A- 4 257 352
US-A- 4 594 965**

(56) Entgegenhaltungen :
**CHEMICAL ABSTRACTS, Band 95, 1981, Seite
453, Zusammenfassung Nr. 165987b, Columbus, Ohio, US; M. VOSYLIENE et al.:
"Reaction of aquatic animals to electric fields.
9. Sensitivity of trout and carp to an electric
current and content of biogenic monoamines
in their tissues during various seasons of the
year", & LICT. TSR MOKSLU AKAD. DARB.,
SER. C 1981, (3), 109-18**

(73) Patentinhaber : **CIBA-GEIGY AG
Klybeckstrasse 141
CH-4002 Basel (CH)**

(72) Erfinder : **Ebner, Guido, Dr.
Bergstrasse 5
CH-5268 Eiken (CH)**
Erfinder : **Schürch, Heinz
Hörnliallee 135
CH-4125 Riehen (CH)**

## Beschreibung

Die vorliegende Erfindung betrifft ein neuartiges Verfahren, welches basierend auf der kurzfristigen Anwendung elektrostatischer Felder zu bleibenden nützlichen und wünschenswerten Eigenschaften bei Fischen führt, die ansonsten überhaupt nicht oder nur mit erheblichem Mehraufwand erzeugbar sind. Durch die Einfachheit der erfindungsgemässen Verfahrensmassnahmen und die signifikanten Resultate erfährt die Aufzucht von Süss- und Salzwasser-Fischen, insbesondere von Speise-, aber auch von Zierfischen, eine drastische Verbesserung.

Bei dem erfindungsgemässen Verfahren handelt es sich im wesentlichen um das kurzzeitige Einbringen von frühen Fischentwicklungsstadien, wie z.B. von Jungfischen oder vorzugsweise von Eiern, vor, während oder nach der Befruchtung, insbesondere aber von Eiern in frühen Zellteilungsphasen, in elektrostatische Felder, unter Ausschluss von fliessenden elektrischen Strömen. Besonders bevorzugt ist die Verwendung von Eiern während oder unmittelbar nach der Befruchtung.

Im Einzelnen werden im Rahmen des erfindungsgemässen Verfahrens mit Süss- oder Salzwasser gefüllte und die Fische bzw. Eier enthaltende Behälter (Aquarien), die vorzugsweise aus elektrisch nicht-leitendem Material (Isolator) bestehen, zwischen die Elektroden eines Kondensators gebracht. An besagte Elektroden wird eine Gleichspannung von einem bis mehreren zehntausend Volt angelegt. Selbstverständlich kann man statt des nichtleitenden Aquarienmaterials auch Elektroden verwenden, die mit einer Isolierbeschichtung versehen sind und diese elektrisch isolierten Kondensatorplatten unmittelbar in die Behälter eintauchen. Wichtig ist nur, dass das als Dielektrikum fungierende Süss- oder Salzwasser nicht in leitender Verbindung mit den Kondensatorelektroden steht. Da in dieser Vorrichtung keine Ströme fliessen, wird auch kein merklicher Energieverlust festgestellt. Demnach ist die Energie auch kein Kostenfaktor bei dieser Erfindung.

Ein weiterer wesentlicher Gesichtspunkt der vorliegenden Erfindung betrifft die Tatsache, dass aufgrund des Fehlens eines elektrischen Stromflusses die chemische Identität des Systems "Fisch" nicht verändert wird.

Das Interesse bei der Untersuchung von Parametern, die eine mittelbare oder unmittelbare Wirkung auf chemisch/physikalische Prozessabläufe bei lebenden Organismen ausüben, konzentrierte sich bisher fast ausschliesslich auf den Einfluss von Temperatur, Druck, elektromagnetischer Strahlung und elektrodynamischen Feldern (Wechselfelder). So offenbart beispielsweise die US-A-4,594,965 ein System Züchtung von Meeresorganismen in einem elektrischen Feld, in dem je nach der Stromquelle Gleichstrom oder Wechselstrom fliesst.

Weit weniger Beachtung fand dagegen beispielsweise die Untersuchung über eventuelle Wechselwirkungen von elektrostatischen Feldern auf die Entwicklung von höheren Organismen, insbesondere von Fischen.

Erst in jüngerer Zeit häufen sich die Berichte über mögliche Auswirkungen von Gravitation und magnetischen Feldern auf biologische Systeme. So konnten beispielsweise Goodman und Henderson [Bioelectromagnetics, 7: 23-29, 1986] Anzeichen dafür finden, dass ein Zusammenhang zwischen elektromagnetischen Feldern und der Transkriptionsrate in biologischem Material besteht, die von dem angelegten EM-Feld positiv beeinflusst wird, im Sinne einer Transkriptionssteigerung.

Die Möglichkeit, dass auch statische Elektrofelder einen Einfluss auf chemisch/physikalische Prozessabläufe in Lebewesen, insbesondere auch in frühen Entwicklungsphasen haben könnten, wurde dagegen bisher offenbar von vornherein ausgeschlossen. Entsprechend existieren bisher auch keine Berichte über eine mögliche Wirkung statischer Elektrofelder auf die Entwicklung von Fischen.

Hingegen sind Untersuchungen beschrieben (C.A. 95: 165 987b; Vosyliene et al.), bei denen Forellen (Salmo iridens) und Karpfen (Cyprinus carpio) über längere Zeiträume Feldstärken von 0.03 - 0.08 V/cm und 0.06 - 0.10 V/cm ausgesetzt waren. Besagte Untersuchungen befassten sich hauptsächlich mit dem Einfluss der angelegten Spannung auf die Hirnentwicklung und Noradrenalinausschüttung in zusätzlicher Abhängigkeit von der Jahreszeit.

Dass Einflüsse statischer Elektrofelder so wenig untersucht sind, mag in erster Linie dadurch begründet erscheinen, dass man gemäss der bisher gängigen Lehrmeinung davon ausging, dass ein statisches Elektrofeld in einem mit Ladungsträgern gefüllten Medium durch die spontane Ausbildung einer elektrischen Doppelschicht abgeschirmt wird und damit in seiner Wirkung inert bleibt.

Diese Lehrmeinung basiert im wesentlichen auf der von C. Gouy und D.L. Chapman aufgestellten Beziehung, nach der die effektive Dicke einer diffusen Doppelschicht für einen Elektrolyten

$$d = \frac{1}{F} \cdot \sqrt{\frac{1000\,\varepsilon\,RT}{4\,\pi\,\Sigma\,c_i z_i^2}}$$

beträgt, worin

d = die Dicke der Doppelschicht
F = die Faradayische Konstante
$\varepsilon$ = die Dielektrizitätskonstante
R = die universelle Gaskonstante

2

T = die absolute Temperatur und

i = Ionenarten der Konzentrationen $c_i$ und der Wertigkeiten $z_i$ bedeuten.

Dieses Vorurteil konnte jetzt im Rahmen der vorliegenden Erfindung in Bezug auf Fische durch Anwendung einfacher Verfahrensmassnahmen überraschenderweise ausgeräumt werden.

Entgegen der oben näher charakterisierten, gängigen Lehrmeinung ist es im Rahmen dieser Erfindung jetzt erstmals gelungen ein Verfahren zu entwickeln, mit dessen Hilfe es möglich ist, basierend auf einer Modifikation chemisch/physikalischer Prozessabläufe aufgrund der Einwirkung eines statischen Elektrofeldes, bei Fischen bleibende wünschenswerte und nützliche Veränderungen zu induzieren.

Dies kann, wie gesagt, am einfachsten dadurch erreicht werden, dass man besagte Jungfische oder aber befruchtete oder unbefruchtete Fischeier in ein statisches Elektrofeld einbringt, sodass die während der Zellteilung und Differenzierung ablaufenden chemisch/physikalischen Prozesse, die mit Hilfe des erfindungsgemässen Verfahrens beeinflusst werden sollen, unter dem Einfluss eines definierten elektrostatischen Feldes unter kontrollierbaren Bedingungen ablaufen.

Gegenstand der vorliegenden Erfindung ist somit ein Verfahren zur Erzeugung wünschenswerter und nützlicher Eigenschaften bei Fischen, das sich dadurch kennzeichnet, dass man

a) frühe Entwicklungsstadien von Fischen in ein statisches Elektrofeld einbringt, sodass die chemisch/ physikalischen Prozesse, die beeinflusst werden sollen, unter dem Einfluss eines definierten elektrostatischen Feldes unter kontrollierbaren Bedingungen ablaufen, ohne dadurch aber die chemische Identität des Systems selbst zu verändern und

b) besagte frühe Entwicklungsstadien von Fischen dort für einen Zeitraum belässt, der für eine stabile Ausbildung der gewünschten Modifikation notwendig ist.

Unter frühen Entwicklungsstadien von Fischen werden hier und im folgenden alle Stadien angefangen vom Ei (Eizelle), vorzugsweise dem befruchteten Ei, über die verschiedenen Embryonalstadien bis hin zum schlupffähigen bzw. geschlüpften Jungfisch verstanden. Es sind dies die Stadien häufiger Zellteilung und Differenzierung, bei denen die elektrostatischen Felder positive und bleibende Resultate erzielen.

Besonders bevorzugt sind dabei Fischeier, die bereits im statischen Feld befruchtet oder aber erst nach der Befruchtung, - am besten unmittelbar danach-, einem elektrostatischen Feld ausgesetzt werden.

Einen günstigen und daher im Rahmen dieser Erfindung bevorzugten Zeitraum für den Verbleib dieser frühen Entwicklungsstadien im elektrostatischen Feld stellt die Zeit der Ausreifung der Eier dar, insbesondere bis zum Schlüpfen. Dieser Zeitraum hängt im allgemeinen von äusseren Bedingungen, wie z.B. der Wassertemperatur, dem $O_2$-Gehalt, etc., insbesondere aber von der Fischart ab.

Danach werden die geschlüpften Jungfische, wie in der Fischzucht üblich, in grössere Becken überführt, wo sie bis zu einem gewünschten Stadium heranwachsen.

Das wirkliche Ueberraschende dabei ist, dass sich diese, auf die zuvor beschriebene Weise vorbehandelten und in normale Zuchtbecken überführten Jungfische im Vergleich zu unbehandelten Kontrollgruppen in signifikanter Weise unterscheiden.

Als erstes stellt man eine wesentlich höhere Schlupfrate bei den behandelten Fischeiern fest, wobei häufig eine Steigerung von 100 bis 300 %, aber auch höhere Werte, auftreten. Diese Jungfische machen überdies einen wesentlich agileren und vitaleren Eindruck als die unbehandelten Vergleichsfische. Ganz signifikant ist ihre ausgesprochen hohe Ueberlebensrate, die sich nicht nur auf die ersten Lebenstage, sondern praktisch auf den gesamten Lebenszyklus erstreckt. Dies wird umso deutlicher, wenn man auf jede medikamentöse Behandlung verzichtet. Dann stellt man nämlich fest, dass die unbehandelte (ohne statisches Elektrofeld) Kontrollgruppe in den ersten Tagen und Wochen durch die nicht künstlich unterdrückte, natürlicherweise vorhandene Population von Krankheitskeimen mindestens doppelt so stark reduziert wird wie die Fische, die eine Behandlung im Elektrofeld erfahren haben. Hinzu kommt, dass die behandelten Fische, bei gleicher Ernährung, wesentlich rascher an Gewicht und Grösse zunehmen und deutlich früher das Erwachsenenstadium erreichen und damit in natürliche Gewässer überführt werden können oder für den Verkauf als Speise- oder Zierfische zu Verfügung stehen.

Im übrigen werden keine nachteiligen Veränderungen bei der Nachkommenschaft dieser behandelten Fische beobachtet. Im Gegenteil, ein gewisser Teil der Vitalität scheint auf die Nachkommenschaft übertragbar zu sein.

Alles in allem sind die erfindungsgemäss behandelten Fische wesentlich vitaler als die unbehandelten Vergleichstiere und erreichen früher das Erwachsenenstadium. Für den Fischzüchter bedeutet dies eine Reduktion hinsichtlich des Medikamenten- und Desinfektionsmittelverbrauchs, bis hin zum totalen Verzicht auf derartige Mittel, eine deutlich effizientere Nutzung der eingesetzten Fischnahrung und eine verkürzte Aufzuchtphase. Dies sind Vorteile, die keine andere gegenwärtig bekannte Massnahme zu erbringen vermag.

Der Mechanismus, der dem erfindungsgemässen Verfahren zugrunde liegt, ist gegenwärtig nicht bekannt und bedarf künftiger Aufklärungsarbeiten.

Insgesamt gesehen führt die Anwendung des erfindungsgemässen Verfahrens überraschenderweise beispielsweise zu einer positiven Veränderung der Entwicklungs- und Wachstumseffizienz, der Morphogenese, möglicherweise der Genexpressionsmuster, der Stressanfälligkeit, der Resistenz gegen Krankheitskeime u.a.m.

Ein wesentlicher Aspekt dieser Erfindung betrifft daher ein Verfahren zur Steigerung der Effizienz von Entwicklung und Wachstum von Süss- und Salzwasserfischen. Besonders bevorzugt ist ein Verfahren zur Steigerung der Effizienz von Entwicklung und Wachstum bei Speisefischen.

Ebenso umfasst von der vorliegenden Erfindung sind die durch Anwendung des erfindungsgemässen Verfahrens resultierenden veränderten Fische selbst, die eine erhöhte Entwicklungs- oder Wachstumseffizienz aufweisen sowie deren Nachkommen, sofern diese noch die neuen und charakteristischen Eigenschaften mindestens eines der verfahrensgemäss veränderten Eltern aufweisen.

Unter einer erhöhten Entwicklungs- und Wachstumseffizienz von Fischen ist im Rahmen dieser Erfindung definitionsgemäss z.B. eine Steigerung der Schlupfraten sowie eine Steigerung der Wachstumsgeschwindigkeit zu verstehen.

Ein weiterer Gegenstand der vorliegenden Erfindung betrifft ein Verfahren, welches die spezifischen Stressreaktionen von Fischen in wünschenswerter und nützlicher Weise modifiziert, insbesondere in dem Sinne, dass die erfindungsgemäss behandelten Tiere robuster sind und rascher das Erwachsenenstadium erreichen.

So ist es beispielsweise möglich, die Anfälligkeit gegenüber Krankheiten durch die Anwendung des erfindungsgemässen Verfahrens drastisch zu verringern und die Fische somit unter Bedingungen zu kultivieren, die normalerweise für ihre Gesundheit kritisch sind und ohne erfindungsgemässe Behandlung eine normale und geregelte Entwicklung nicht zulassen.

Ebenso umfasst von der vorliegenden Erfindung sind daher Fische, deren spezifische Reaktionen auf bestimmte Stressparameter durch die Anwendung des erfindungsgemässen Verfahrens in einer gewünschten und nützlichen Art und Weise modifiziert sind, insbesondere solche, die eine erhöhte Resistenz gegenüber Krankheitskeimen aufweisen.

Besonders bevorzugt im Rahmen dieser Erfindung ist ein Verfahren zur Vitalisierung von Fischen, das durch die folgenden Verfahrensmassnahmen gekennzeichnet ist:

a) Einbringen von befruchteten Fischeiern in ein statisches Elektrofeld unter Ausschluss von Stromfluss oder Befruchtung der Eier in besagtem Feld,

b) Einstellen der Feldstärke auf Werte zwischen 1 V/cm und $10^5$ V/cm und

c) Aufrechterhaltung des statischen Elektrofeldes bis zum Schlüpfen oder zur Ausreifung der Jungfische.

Selbstredend kann der Befruchtungsvorgang auch in der erfindungsgemässen Apparatur (Aquarium mit Elektroden) bei bereits angelegter Spannung durchgeführt werden.

Im Rahmen der vorliegenden Erfindung wird das statische Elektrofeld vorzugsweise zwischen den elektrisch isolierten Platten eines Kondensators aufgebaut.

Die elektrische Feldstärke des statischen Elektrofeldes ist durch die folgende Beziehung gegeben:

$$E = \frac{U}{d}$$

worin U die Potentialdifferenz (Spannung) zwischen den Kondensatorplatten und d den Plattenabstand bedeuten.

Die elektrische Spannung U wird durch einen Hochspannungsgenerator erzeugt. Es können beliebige Hochspannungsgeneratoren im Rahmen dieser Erfindung verwendet werden; bevorzugt sind Hochspannungsgeneratoren, die auf dem Transformatorprinzip mit Gleichrichter beruhen.

Die im Rahmen dieser Erfindung bevorzugte elektrische Spannung beträgt zwischen 1,0 V (Volt) und $10^5$ (V) Volt.

Für die Anwendung des erfindungsgemässen Verfahrens auf Fischeier verwendet man i.a. Spannungen von 1 V bis 20'000 V, insbesondere von 100 V bis 10'000 V. Ganz besonders bevorzugt ist eine Spannung von 500 V bis 3'000 V.

Der Plattenabstand des Kondensators richtet sich nach den Dimensionen des Behälters (Aquariums) und beträgt z.B. zwischen 0,01 mm und 1 m, vorzugsweise aber zwischen 1 cm und 10 cm.

In der Praxis wird normalerweise die Feldstärke des statischen Feldes bei gegebenem Plattenabstand durch die Höhe der Spannung an einem Hochspannungsgenerator reguliert.

Im Rahmen der vorliegenden Erfindung betragen die verwendeten Feldstärkewerte vorzugsweise zwischen 1 V/cm und 10'000 V/cm, besonders bevorzugt zwischen 50 V/cm und 5'000 V/cm, insbesondere aber zwischen 500 V/cm und 1'000 V/cm.

Ganz besonders bevorzugt für einen Einsatz in dem erfindungsgemässen Verfahren ist biologisches Material, das eine hohe Teilungsaktivität aufweist und/oder noch wenig differenziert ist, wie z.B. teilungsaktive Zellen. Im einzelnen erwähnt seien an dieser Stelle befruchtete oder unbefruchtete Eier sowie frühe Embryonal-Stadien bis hin zum schlupffähigen Jungfisch.

Die Befruchtungs- und Schlupfrate bei Fischen kann dabei unter dem Einfluss eines statischen Elektrofeldes signifikant gesteigert werden.

Alle diese beispielhaften Aufzählungen dienen nur der Verdeutlichung der vorliegenden Erfindung und schränken den Erfindungsgegenstand in keiner Weise ein.

Besonders bevorzugt im Rahmen dieser Erfindung ist ein Verfahren, bei dem die Entwicklung der befruchteten Eier zum Jungfisch in einem statischen Elektrofeld erfolgt, wobei die Feldstärkewerte vorzugsweise zwischen 10 V/cm und 3'000 V/cm liegen können. Besonders bevorzugt sind Feldstärkewerte von 500 V/cm bis 1'500 V/cm, insbesondere von 500 V/cm bis 1'000 V/cm.

Im einzelnen handelt es sich dabei um ein Verfahren zur Steigerung der Wachstums- und Entwicklungseffizienz bei Fischen, das sich dadurch kennzeichnet, dass man die Befruchtungs-, Schlupf- und Ueberlebensrate sowie das Wachstum der Fische erhöht, indem man

a) Fischeier mit männlichem Samen versetzt,

b) diese in Brutzellen einbringt, in denen ein statisches Elektrofeld aufgebaut wird,

c) Feldstärkewerte für das statische Elektrofeld zwischen 10 V/cm und 3'000 V/cm einstellt,

d) die Jungfische nach dem Schlüpfen aus dem Einflussbereich des statischen Elektrofeldes herausnimmt und

e) besagte Jungfische nach an sich bekannten Methoden aufzieht.

Die Behandlung der Fische im statischen Elektrofeld erfolgt im Rahmen dieser Erfindung vorzugsweise in speziellen Brutzellen, die im Boden sowie im Deckel wasserdicht und vor allem nicht-leitend eingegossene Elektroden enthalten, die mit einer Quelle zur Erzeugung einer hohen Gleichspannung leitend verbunden sind und mit deren Hilfe ein statisches Elektrofeld im Innern der Brutzelle aufgebaut werden kann. Selbstverständlich kann auch jeder beliebige andere Versuchsaufbau für die Behandlung der Fische verwendet werden, sofern dieser die vorgegebenen Rahmenbedingungen erfüllt.

Nach dem Schlüpfen der Jungfische wird das Elektrofeld abgeschaltet und die weitere Aufzucht der Jungfische nach allgemein üblichen Verfahren ohne den Einfluss eines Elektrofeldes fortgeführt.

Wider alle Theorie und daher äusserst überraschend zeigt es sich, dass die im Elektrofeld geschlüpften Jungfische gegenüber den Kontrollen über eine wesentlich erhöhte Befruchtungs- und Schlupfrate hinaus weitere vorteilhafte Eigenschaften aufweisen, die nunmehr auch nach Wegfall des Feldes zur Ausprägung gelangen und sich auf die weitere Entwicklung der Fische auswirken.

Dazu gehören z.B. eine starke Reduktion der Abgänge an Jungfischen sowie eine erhöhte Wachstumsgeschwindigkeit im Vergleich zu den Kontrollen. Darüber hinaus zeigen die behandelten Versuchstiere eine deutlich gesteigerte Vitalität im Vergleich zu den Kontrolltieren.

Ein weiterer Gegenstand der vorliegenden Erfindung betrifft die Anwendung des erfindungsgemässen Verfahrens zur Modifikation spezifischer Stressreaktionen von Fischen.

Besonders bevorzugte Anwendungsbereiche betreffen die Erhöhung der Stressresistenz z.B. gegenüber bestimmten Umweltfaktoren, wie erhöhten Salzkonzentrationen im Kulturmedium, Limitierung essentieller Nährstoffe, Limitierung von Licht und/oder $O_2$-Versorgung, Akzeptanz von Verschmutzungen u.a.m.

Einer der häufigsten Stressfaktoren in der belebten Umwelt betrifft den Mangelstress, wobei ein oder aber mehrere Faktoren gleichzeitig limitierend wirken können.

Mangelstress tritt auf, sobald einer oder mehrere der für ein optimales Wachstum oder eine optimale Entwicklung notwendigen Faktoren, wie Licht, $O_2/CO_2$-Versorgung, Nährstoffangebot, Vitamine etc. suboptimale Werte erreicht.

Dies führt dazu, dass der betreffende Organismus bestimmte, für eine optimale Entwicklung und ein optimales Wachstum notwendige Syntheseleistungen nicht mehr in vollem Umfang aufrecht erhalten kann, was zunächst zu einer Verlangsamung des Wachstums führt. Hält dieser Mangelstress über einen längeren Zeitraum an, so führt dies schliesslich zu einer Beeinträchtigung essentieller Lebensfunktionen, was schliesslich in der Regel zu einem frühzeitigen Einsetzen der Seneszenz und damit letztlich zum Absterben des betroffen Organismus führt.

Nicht nur ein Mangel, sondern auch ein Ueberangebot an bestimmten kritischen Faktoren kann zur Auslösung von Stressreaktionen führen. Erhöhte Salzkonzentration im Nährmedium beispielsweise führt zu einer Erhöhung des osmotischen Wertes im Medium und infolgedessen zu einem Flüssigkeitsverlust der darin befindlichen Zellen infolge einer einsetzenden Osmose und damit zum Schrumpfen der Zellen. Der Flüssigkeitsverlust kann in der Regel durch ausgleichende Massnahmen der betroffenen Organismen in gewissem Rahmen kompensiert werden. Ist jedoch ein kritischer Schwellenwert überschritten, so führt dies zum Absterben der betroffenen Zellen.

Das erfindungsgemässe Verfahren ist prinzipiell auf alle Arten von Fischen anwendbar, insbesondere aber auf Arten, die in kommerziellem Massstab gezüchtet werden.

Besondere Bedeutung erlangt dabei die Behandlung von Speisefischen, da zu erwarten ist, dass die Produktionskosten mit Hilfe des erfindungsgemässen Verfahrens drastisch gesenkt werden können.

Zur Illustration der eher allgemeinen Beschreibung sowie zum besseren Verständnis der vorliegenden Erfindung soll nunmehr auf spezifische Ausführungsbeispiele Bezug genommen werden, die keinen limitierenden Charakter haben, es sei denn, es wird speziell darauf hingewiesen. Das gleiche gilt auch für alle beispielhaften Aufzählungen, die in der vorangegangenen Beschreibung enthalten sind.

Nichtlimitierte Ausführungsbeispiele

Aufbau des statischen Elektrofeldes - Versuchsaufbau (nachfolgend zitiert als "Versuchsaufbau")

Alle im folgenden beschriebenen Versuche werden in statischen Elektrofeldern durchgeführt, die zwischen den Platten eines Kondensators aufgebaut werden.

Die elektrische Feldstärke ist durch die Beziehung $E = \dfrac{W}{d}$ gegeben, worin

U die Spannung (Potentialdifferenz) zwischen den Kondensatorplatten und
d den Plattenabstand des Kondensators
bedeuten.

Die Spannung wird mit Hilfe eines Hochspannungsgenerators erzeugt, der auf dem Transformatorprinzip mit Gleichrichter basiert, wobei in der Regel Spannungen zwischen 500 Volt und 12'000 Volt verwendet werden.

Der Plattenabstand des Kondensators richtet sich nach den Dimensionen der in den einzelnen Versuchen verwendeten Probengefässe. In der Regel werden die variablen Parameter U und d so gewählt, dass das statische Elektrofeld Feldstärkewerte zwischen 250 V/cm und 1500 V/cm aufweist.

Für die Versuche mit Fischen, nachfolgend mit Forellen, wurde eine von diesem allgemeinen Schema etwas abweichende Versuchsanordnung gewählt, die in Beispiel 1 im einzelnen beschrieben ist.

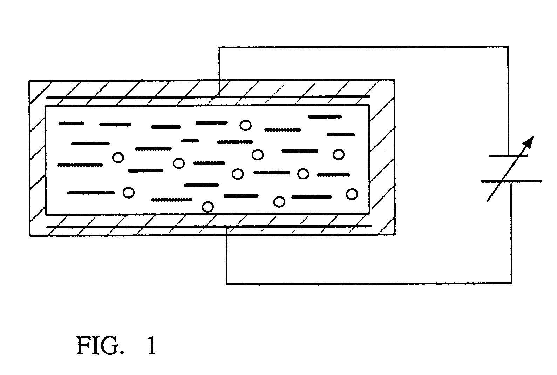

Die vorliegende Erfindung betrifft auch eine Vorrichtung zur Behandlung von frühen Fischentwicklungsstadien mit statischen Elektrofeldern mit

a) einem zur Aufnahme der frühen Fischentwicklungsstadien in wässrigem Medium geeigneten Behälter und

b) einer elektrischen Spannungsquelle sowie einer mit dieser verbundenen Elektrodenanordnung zur Erzeugung eines elektrischen Felds in wenigstens einem Teil des vom Behälter umschlossenen Volumens,

dadurch gekennzeichnet, dass die Elektrodenanordnung gegenüber dem im Behälter befindlichen Medium elektrisch isoliert und vorzugsweise als Platten eines Kondensators ausgebildet ist, und dass die Spannungsquelle eine Gleichspannungsquelle ist. (siehe Fig. 1)

Beispiel 1: Auswirkung eines statischen Elektrofeldes auf die Entwicklung von Fischen

Neben dem Keimungsverhalten von Pflanzensamen kann mit Hilfe eines statischen Elektrofeldes auch die Entwicklung tierischer Organismen positiv beeinflusst werden, was im folgenden am Beispiel der Regenbogenforelle demonstriert wird.

1.1. Versuchsaufbau

Je 1000 Fischeier von Forellen werden in einem Becken mit männlichem Samen versetzt und sofort in Brutzellen eingetragen. Die Brutzellen bestehen aus einem Plexiglasgefäss mit folgenden Ausmassen: 31,5 x 28,5 x 4,5 cm und 1 l Inhalt. Im Boden sowie im Deckel sind luft- und wasserdicht verschlossen Aluminiumelektroden eingegossen.

Die Schalen werden an eine Quellwasserleitung von 10-12°C angeschlossen und die Elektroden mit einem Hochspannungsgenerator (FUG HCN 14-12500; Fa. Weter, Pfaffhausen, CH) leitend verbunden. Die angelegte Spannung beträgt 2150 Volt bei einem Plattenabstand von 3 cm, wodurch eine Feldstärke von 716 V/cm zwischen den Elektroden entsteht. In dieser Anlage erfolgt die Befruchtung sowie die Ausreifung der Eier. Nach ca. 4 Wochen ist das Augenstadium erreicht, nach 8 Wochen schlüpft die Brut.

Nach dem Schlüpfen wird die Brut in laminar durchströmte Becken von 50 x 50 x 15 cm übertragen und das Elektrofeld entfernt, sodass die weitere Entwicklung der Jungfische ohne den Einfluss des Feldes erfolgt.

1.2. Ergebnisse

Tabelle 1 zeigt, dass die Anzahl der unter dem Einfluss des statischen Elektrofeldes geschlüpften Jungfische deutlich höher liegt als bei der Kontrolle.

Die Abgänge unmittelbar nach dem Schlüpfen sowie nach 5 Wochen konnten dagegen mit Hilfe des Feldes auf ca. die Hälfte reduziert werden im Vergleich zu den Kontrollen.

Darüber hinaus lässt sich auch ein wachstumssteigernder Effekt des Feldes nachweisen. Die Grösse der Jungfische 10 Wochen nach dem Schlüpfen liegt bei den unter dem Einfluss des statischen Elektrofeldes geschlüpften Jungfischen zwischen 30 % bis 40 % höher als bei den Kontrollfischen (vgl. Tabelle 1).

Tabelle 1: Vergleich der Wachstums- und Entwicklungseffizienz von Regenbogenforellen mit und ohne Elektrofeld

|  | ohne Feld | mit Feld | |
| --- | --- | --- | --- |
|  |  | Becken 1 | Becken 2 |
| geschlüpfte Jungfische | 140 | 244 | 469 |
| Abgänge nach Schlüpfen | 37 | 14 | 19 |
| Abgänge nach 5 Wochen | 10 | 5 | 6 |
| Grösse nach 10 Wochen | 2,6 cm | 3,4 cm | 3,6 cm |

Die im Feld bebrüteten Fische zeigen eine deutlich erhöhte Vitalität.

**Patentansprüche**

1. Verfahren zur Steigerung der Effizienz von Entwicklung und Wachstum sowie zur Modifikation spezifischer Stressreaktionen in Fischen, dadurch gekennzeichnet, dass man
   a) ein statisches Elektrofeld zwischen Kondensatorplatten erzeugt, die gegen Wasser als dielektrischem Medium isoliert sind, wodurch ein Stromfluss verhindert wird;
   b) frühe Entwicklungsstadien von Fischen in besagtes Elektrofeld einbringt, sodass die chemisch/physikalischen Prozesse, die beeinflusst werden sollen, unter dem Einfluss eines definierten elektrostatischen Feldes unter kontrollierbaren Bedingungen ablaufen, ohne dadurch aber die chemische Identität des Systems "Fisch" selbst zu verändern und
   c) besagte frühe Entwicklungsstadien dort für einen Zeitraum belässt, der für eine stabile Ausbildung der gewünschten Modifikation notwendig ist.

2. Verfahren nach Anspruch 1, dadurch gekennzeichnet, dass man befruchtete Fischeier in das statische Elektrofeld einbringt.

3. Verfahren nach Anspruch 1, dadurch gekennzeichnet, dass man Fischeier im statischen Elektrofeld befruchtet.

4. Verfahren nach Anspruch 2, dadurch gekennzeichnet, dass man die befruchteten Eier bis zum Ausreifen im statischen Elektrofeld belässt.

5. Verfahren nach Anspruch 3, dadurch gekennzeichnet, dass man die befruchteten Eier bis zum Schlüpfen der Jungfische im starischen Elektrofeld belässt.

6. Verfahren nach Anspruch 1 zur Steigerung der Effizienz von Entwicklung und Wachstum von Süss- und Salzwasserfischen.

7. Verfahren nach Anspruch 6 zur Steigerung der Schlupfraten.

8. Verfahren zur Vitalisierung von Fischen, gekennzeichnet durch die Massnahmen:
   a) Einbringen von befruchteten Eiern in ein statisches Elektrofeld unter Ausschluss von Stromfluss oder Befruchtung der Eier in besagtem Feld;

7

b) Einstellen der Feldstärke auf Werte zwischen 1,0 V/cm und $10^5$ V/cm; und

c) Aufrechterhaltung des statischen Elektrofeldes bis zur Ausreifung oder Schlüpfung der Jungfische.

9. Verfahren nach Anspruch 8, dadurch gekennzeichnet, dass man zur Erzeugung des statischen Elektrofeldes Kondensatorplatten verwendet, die in keiner leitenden Verbindung zu dem als Dielektrikum fungierenden Wasser stehen.

10. Verfahren zur Steigerung der Wachstums- und Entwicklungseffizienz bei Fischen, durch Erhöhung der Befruchtungs-, Schlupf- und Ueberlebensrate sowie des Wachstums der Fische dadurch gekennzeichnet, daß man

a) Fischeier mit männlichem Samen versetzt,

b) diese in Brutzellen in einem statischen Elektrofeld einbringt, das zwischen Kondensatorplatten, die gegen Wasser als dielektrischem Medium isoliert sind, erzeugt wird, wodurch ein Stromfluss verhindert wird;

c) Feldstärkewerte für das starische Elektrofeld zwischen 10 V/cm und 10'000 V/cm einstellt,

d) die Jungfische nach dem Schlüpfen aus dem Einflussbereich des statischen Elektrofeldes herausnimmt und

e) besagte Jungfische nach an sich bekannten Methoden aufzieht.

11. Verfahren gemäss Anspruch 10, dadurch gekennzeichnet, dass die Feldstärkewerte zwischen 10 V/cm und 3'000 V/cm betragen.

12. Verfahren gemäss Anspruch 10, dadurch gekennzeichnet, dass die Feldstärkewerte zwischen 500 V/cm und 1'000 V/cm betragen.

13. Verfahren gemäss Anspruch 10, dadurch gekennzeichnet, dass es sich um Forellen handelt.

14. Vorrichtung zur Behandlung von frühen Fischentwicklungsstadien mit starischen Elektrofeldern mit a) einem zur Aufnahme der frühen Fischentwicklungsstadien in wässrigem Medium geeigneten Behälter und b) einer elektrischen Spannungsquelle sowie einer mit dieser verbundenen Elektrodenanordnung zur Erzeugung eines elektrischen Felds in wenigstens einem Teil des vom Behälter umschlossenen Volumens, dadurch gekennzeichnet, dass die Elektrodenanordnung gegenüber dem im Behälter befindlichen Medium elektrisch isoliert und vorzugsweise als Platten eines Kondensators ausgebildet ist, und dass die Spannungsquelle eine Gleichspannungsquelle ist.

## Claims

1. A method of enhancing the efficiency of the development and growth of fish and of modifying specific stress reactions in fish, which comprises

a) producing an electrostatic field between capacitor plates that are insulated against water as the dielectric medium, which prevents the flow of current;

b) exposing early development stages of fish to the said electrostatic field, such that the physico-chemical processes which it is desired to modify take place under the influence of a defined electrostatic field under controlled conditions, but without thereby changing the chemical identity of the "fish" system itself, and

c) keeping said early development stages in said electrostatic field for a period of time necessary for a stable development of the desired modification.

2. A method according to claim 1, which comprises exposing fertilised fish eggs to the electrostatic field.

3. A method according to claim 1, which comprises fertilising fish eggs in the electrostatic field.

4. A method according to claim 2, which comprises leaving the fertilised eggs to ripen in the electrostatic field.

5. A method according to claim 3, which comprises leaving the fertilised eggs in the electrostatic field until the juvenile fish hatch.

**6.** A method according to claim 1 for enhancing the efficiency of development and growth of freshwater and saltwater fish.

**7.** A method according to claim 6 for enhancing the hatching rates.

**8.** A method of vitalising fish, which comprises the steps of
a) exposing fertilised eggs to an electrostatic field, without flow of current, or fertilising eggs in said field;
b) adjusting the field strength to values of from 1.0 V/cm to $10^5$ V/cm; and
c) maintaining the electrostatic field until the juvenile fish hatch or mature.

**9.** A method according to claim 8, wherein capacitor plates are used to produce the electrostatic field, which plates are insulated against the water acting as dielectric medium.

**10.** A method of enhancing the growth and development efficiency of fish by enhancing the fertilisation, hatching and survival rates as well as the growth of fish, which method comprises
a) treating fish eggs with male sperm,
b) placing the treated eggs in incubators in an electrostatic field that is produced between capacitor plates that are insulated against water as the dielectric medium, which prevents the flow of current,
c) providing field strengths for the electrostatic field of 10 V/cm to 10 000 V/cm,
d) removing the juvenile fish, after hatching, from the sphere of influence of the electrostatic field, and
e) rearing said juvenile fish by methods which are known per se.

**11.** A method according to claim 10, wherein the field strengths are from 10 V/cm to 3 000 V/cm.

**12.** A method according to claim 10, wherein the field strengths are from 500 V/cm to 1 000 V/cm.

**13.** A method according to claim 10, wherein the fish are trout.

**14.** An apparatus for the treatment of early development stages of fish with electrostatic fields, said apparatus comprising
a) a tank suitable for holding the early development stages of fish in aqueous medium, and
b) a source of voltage and, connected therewith, an arrangement of electrodes suitable for producing an electric field in at least a part of the volume surrounded by the tank, which is characterised in that the arrangement of electrodes is electrically insulated against the medium contained in the tank and is preferably in the form of capacitor plates, and that the source of voltage is a source of direct-current voltage.

**Revendications**

**1.** Procédé d'accroissement de l'efficacité du développement et de la croissance ainsi que de modification de réactions spécifiques au stress de poissons, caractérisé en ce que
a) on produit un champ électrostatique entre des plaques de condensateur qui sont isolées de l'eau qui forme le milieu diélectrique, ce qui empêche un flux de courant ;
b) on place des stades précoces de développement de poissons dans ledit champ électrique, de manière que les processus chimio-physiques qui doivent être modifiés se déroulent sous l'influence d'un champ électrostatique défini dans des conditions contrôlées, sans modifier toutefois ainsi l'identité chimique du système "poisson" lui-même et
c) on y laisse lesdits stades précoces de développement pendant une période qui est nécessaire pour une production stable de la modification souhaitée.

**2.** Procédé selon la revendication 1, caractérisé en ce qu'on place des oeufs fécondés de poisson dans le champ électrostatique.

**3.** Procédé selon la revendication 1, caractérisé en ce qu'on féconde les oeufs de poisson dans le champ électrostatique.

**4.** Procédé selon la revendication 2, caractérisé en ce qu'on laisse les oeufs fécondés dans le champ électrostatique jusqu'à maturation.

5. Procédé selon la revendication 3, caractérisé en ce qu'on laisse les oeufs fécondés dans le champ électrostatique jusqu'à éclosion des jeunes poissons.

6. Procédé selon la revendication 1 pour l'accroissement de l'efficacité du développement et de la croissance de poissons d'eau douce et d'eau salée.

7. Procédé selon la revendication 6 pour l'accroissement des taux d'éclosion.

8. Procédé de vitalisation de poissons, caractérisé par les mesures suivantes :
   a) mise en place d'oeufs fécondés dans un champ électrostatique à l'exclusion de flux de courant ou fécondation des oeufs dans ledit champ ;
   b) réglage de l'intensité du champ à des valeurs comprises entre 1,0 V/cm et $10^5$ V/cm ; et
   c) maintien du champ électrostatique jusqu'à maturation ou éclosion des jeunes poissons.

9. Procédé selon la revendication 8, caractérisé en ce qu'on utilise pour produire le champ électrostatique des plaques de condensateur qui ne sont pas en liaison conductrice avec l'eau assumant la fonction de diélectrique.

10. Procédé d'accroissement de l'efficacité de la croissance et du développement de poissons, par élévation des taux de fécondation, d'éclosion et de survie ainsi que de la croissance des poissons, caractérisé en ce que
    a) on mélange des oeufs de poissons avec des semences mâles,
    b) on les place dans des cellules d'incubation à l'intérieur d'un champ électrostatique qui est produit entre des plaques de condensateur qui sont isolées de l'eau constituant le milieu diélectrique de manière à empêcher un flux de courant ;
    c) on règle les intensités du champ électrostatique à des valeurs comprises entre 10 V/cm et 10.000 V/cm,
    d) on extrait de la zone d'influence du champ électrostatique les jeunes poissons après leur éclosion et
    e) on élève lesdits jeunes poissons d'après des méthodes connues en soi.

11. Procédé selon la revendication 10, caractérisé en ce que les intensité du champ sont comprises entre 10 V/cm et 3.000 V/cm.

12. Procédé selon la revendication 10, caractérisé en ce que les intensités du champ sont comprises entre 500 V/cm et 1.000 V/cm.

13. Procédé selon la revendication 10, caractérisé en ce qu'il s'agit de truites.

14. Dispositif de traitement de stades précoces de développement de poissons par des champs électrostatiques, comprenant a) un récipient qui convient pour loger les stades précoces de développement de poissons dans un milieu aqueux et b) une source de tension électrique ainsi qu'un montage d'électrodes connecté à celle-ci pour produire un champ électrique dans au moins une partie du volume renfermé par le récipient, caractérisé en ce que le montage d'électrodes est isolé électriquement du milieu se trouvant dans le récipient et a de préférence la forme de plaques d'un condensateur et en ce que la source de tension est une source de tension continue.

FIG. 1